# EUROPEAN PATENT APPLICATION

(11) **EP 2 612 616 A1**
(43) Date of publication of application: **10.07.2013**
(21) Application number: 13150289.0
(22) Date of filing: 04.01.2013
(51) Int. Cl.: A61B 19/00

(54) **Surgical robot and method for controlling the same**

(30) Priority: 06.01.2012 KR 20120001785
(71) Applicant: Samsung Electronics Co., Ltd, Gyeonggi-do 443-742 (KR)
(72) Inventor: Roh, Chang Hyun, Seoul (KR)
(74) Representative: Walaski, Jan Filip

(57) **Abstract**

A surgical robot includes a console and a manipulator assembly. The manipulator assembly includes at least one arm having a plurality of links and a motor provided between links adjacent to each other among the plurality of links, and a control unit configured to determine whether a mode is changed between a teleoperation mode and a manual mode. The control unit sets output data of the motor provided before the mode is changed as input data of the motor provided after the mode is changed, if it is determined that the mode is changed between the teleoperation mode and the manual mode, thereby preventing the vibration and the rapid change of the posture from occurring at the arm of the surgical robot during the change of the mode between the manual mode and the teleoperation mode, thereby increasing stability of the surgical robot.

## Description

The disclosure relates to a surgical robot configured to control a motor to improve the safety of performing an operation using the surgical robot, and a control method thereof.

Generally, a surgical robot is a robot configured to perform a treatment or a surgery on an affected area by using a surgical instrument according to a command of a user.

Some types of surgery performed by a surgical robot include minimal invasive surgery in which a size of an affected area is minimal, e.g., a laparoscopic surgery, a da Vinci robot surgery, etc.

Here, minimal invasive surgery refers to surgery in which a few small incisions are made, unlike open surgery having an abdomen entirely open to perform surgery. In minimal invasive surgery, the abdomen is filled with gas to create a surgical space, and a laparoscope and a manipulator are inserted through the incisions such that surgery is performed using a surgical manipulator while observing an internal image of the abdomen.

The minimal invasive surgery generally involves less post-surgical pain while enabling an early recovery of intestinal movement and of the ability to ingest food earlier relative to open or invasive surgeries. In addition, minimal invasive surgery requires shorter length of hospitalization, and thereby a return to a normal condition is faster. Furthermore, since an area of an incision from the minimal invasive surgery is small, an aesthetic effect is superior. Thus, minimal invasive surgery is being applied in numerous types of surgeries, including gall bladder removal surgery, prostate cancer surgery, hernia correction surgery, etc., and is increasingly being used in the medical field.

A minimal invasive surgery, however, includes difficulty in controlling a surgical instrument and in moving an instrument through an incision. In addition, with respect to minimal invasive surgery, the position displayed through an image to a user, when compared to the actual position inside an abdomen, is reversed both vertically and horizontally. Therefore, a skilled physician as well as medical staff are needed.

The weaknesses of minimal invasive surgery may be overcome in part by performing surgery using a da Vinci robot. Here, a da Vinci robot is configured to deliver a dimensional image, which is expanded by 10 to 15 times in size without a vertical/horizontal reversal, to a user, and is also configured to deliver the movement of a user precisely to a robot arm and a surgical instrument. Generally, a da Vinci robot refers to a conventional robotic surgical system used to perform minimally invasive surgeries.

The surgical robot as such operates in such a way that a laparoscope and a surgical manipulator are inserted through an incision to secure a space for surgery and perform a treatment or surgery of an affected area by moving a surgical manipulator inside the secured space.

At this time, after the insertion of a surgical manipulator is completed, the movement of the surgical manipulator is controlled. After a surgical procedure is completed, the movement of a surgical manipulator is stopped and the surgical manipulator is withdrawn outside of the affected area.

In this case, an operation mode of the surgical manipulator is changed, and with the change of the operation mode, an inconsistency of a control operation or a discontinuity of an output takes place, so that a momentary vibration at the surgical manipulator or an abrupt change in a posture of the surgical manipulator is caused. These vibrations or abrupt changes in the posture of the surgical manipulator may result in damaging an affected area or a portion of a body surrounding an incision.

Therefore, it is an aspect of the present disclosure to provide a surgical robot, when a control mode is changed from a manual mode to a teleoperation mode, capable of setting an angle at a point of time when the manual mode is completed to an initial angle the teleoperation mode starts, and a control method thereof.

It is another aspect of the present disclosure to provide a surgical robot, when a control mode is changed from a teleoperation mode to a manual mode, capable of setting a torque at a point of time when the teleoperation mode is completed to an initial torque of the manual mode, and a control method thereof.

Additional aspects of the disclosure will be set forth in part in the description which follows and, in part, will be apparent from the description, or may be learned by practice of the disclosure.

In accordance with one aspect of the present disclosure, with reference to a surgical robot having a console and a manipulator assembly, the manipulator assembly includes at least one arm and a control unit. The arm may have a plurality of links and a motor provided between links adjacent to each other among the plurality of links. The control unit may be configured to determine whether a mode is changed between a teleoperation mode controlling the motor based on a command transmitted from the console and a manual mode controlling the motor based on an external force, set an output data of the motor provided before the mode is changed as input data of the motor provided after the mode is changed, if determined that the mode is changed between the teleoperation mode and the manual mode, and controls the motor based on the input data, which is set, at the time the mode is changed.

The manipulator assembly may further include a torque detection unit configured to detect the external force applied to the arm. The control unit may be configured to control or switch to the manual mode if the external force is detected.

The control unit, in a case of controlling the motor at the manual mode, may be configured to calculate a torque corresponding to the external force, and control a current applied to the motor based on the torque calculated.

The manipulator assembly may further include an angle detection unit configured to detect an angle of the motor. The control unit, when the command is transmitted from the console during the manual mode, may be configured to change the manual mode to the teleoperation mode, and set the angle detected as an initial angle of the teleoperation mode.

The control unit may be configured to control a change of the angle of the motor from the initial angle to an angle corresponding to the command transmitted from the console.

The control unit, in a case of controlling the motor in the teleoperation mode, may be configured to generate a position command based on the command transmitted from the console, calculate an angle corresponding to the position command generated, and control a current applied to the motor based on the angle calculated.

The control unit may be configured to control a speed of the motor when the current applied to the motor is controlled based on the angle calculated.

The control unit, if determined that the mode is changed from the teleoperation mode to the manual mode, may be configured to check a torque of the motor provided when the motor is controlled in the teleoperation mode, and sets the torque checked as an initial torque of the manual mode.

The manipulator assembly may further include a torque detection unit configured to detect the external force applied to the arm. The control unit may be configured to calculate a torque corresponding to the external force when the mode is changed from the teleoperation mode to the manual mode, and controls the torque of the motor from the initial torque to the torque calculated.

The control unit may be configured to control the torque of the motor by performing an interpolation calculation.

The control unit may include a command generating unit, a position adjusting unit, a current producing unit, and an electric power converting unit. The command generating unit may be configured to generate the position command and torque command. The position adjusting unit may be configured to adjust the angle of the motor in response to the position command generated. The current producing unit may be configured to calculate a current used to follow a torque corresponding to the generated torque command and a current used to adjust the angle of the motor. The electrical power converting unit may be configured to adjust a pulse applied to the motor based on the current calculated.

In accordance with another aspect of the present disclosure, a method of controlling a surgical robot having a console and a manipulator assembly is as follows. An external force applied to an arm provided at the manipulator assembly is detected. A manual mode configured to control a torque of the motor based on the external force detected may be performed. Whether a command is transmitted from the console may be determined. A point in time to change from the manual mode to the teleoperation mode may be determined when the command is transmitted from the console. An angle of the motor may be detected. The angle detected may be set as an initial angle of the teleoperation mode. A change of the angle of the motor may be controlled from the initial angle to an angle corresponding to the command transmitted. The teleoperation mode may be controlled.

The performing of the manual mode may be performed as follows. A torque command corresponding to the external force may be generated. A current to follow the torque command generate may be calculated. A pulse width of the current is adjusted and a current reflecting the adjusted pulse width may be applied to the motor.

The performing of the teleoperation mode may be performed as follows. A position command based on the command transmitted from the console may be generated. An angle corresponding to the position command generated may be calculated. A current corresponding to the angle calculated may be calculated. A pulse width of the current may be adjusted and a current reflecting the adjusted pulse width may be applied to the motor.

The method is achieved by further controlling a speed of the motor.

The method is achieved by further performing as follows. A point in time to change from the teleoperation mode to the manual mode may be determined when the external force applied to the arm is detected during the execution of the teleoperation mode. The torque of the motor at the time of the completion of the execution of the teleoperation mode may be checked. The torque checked may be set as an initial torque of the manual mode. The torque of the motor may be controlled from the initial torque to a torque corresponding to the external force.

The controlling of the torque of the motor may include executing an interpolation operation.

In accordance with another aspect of the present disclosure a surgical robot includes a console and a manipulator assembly. The manipulator assembly includes at least one arm having a plurality of links and a motor provided between links adjacent to one another among the plurality of links, and a control unit. The control unit may be configured to determine when to switch between a first mode and a second mode. When it is determined to perform a switch from the first mode to the second mode, a first parameter from the first mode used to control the motor is calculated, and the calculated first parameter is used as an initial parameter to control the motor when the mode is switched to the second mode. If the first mode is a teleoperation mode, and the second mode is a manual mode, the first parameter may include a torque value of the motor. If the first mode is a manual mode and the second mode is a teleoperation mode, the first parameter may include an angle of the motor.

As described, an occurrence of a vibration with respect to the arm of the surgical robot may be prevented at the time of changing the mode between the manual mode and the teleoperation mode, and the abrupt change of the posture of the surgical robot may be restrained, thereby increasing the stability and safety of the surgical robot.

These and/or other aspects of the disclosure will become apparent and more readily appreciated from the following description of the embodiments, taken in conjunction with the accompanying drawings in which:
FIG. 1 is a view illustrating a manipulator assembly provided at a surgical robot in accordance with an embodiment of the present disclosure.
FIG. 2 is a view illustrating an arm of a manipulator assembly provided at the surgical robot in accordance with the embodiment of the present disclosure.
FIG. 3 is a view illustrating a surgical instrument provided at the surgical robot in accordance with the embodiment of the present disclosure.
FIG. 4 is a control block diagram illustrating the surgical robot in accordance with the embodiment of the present disclosure.
FIG. 5 is a detailed control block diagram illustrating the surgical robot in accordance with the embodiment of the present disclosure.
FIG. 6 is a view illustrating a manual mode of the surgical robot in accordance with the embodiment of the present disclosure.
FIG. 7 is a view illustrating a data change in a case of a mode change of the surgical robot in accordance with the embodiment of the present disclosure.
FIGS. 8 to 9 are control flow charts illustrating the surgical robot in accordance with the embodiment of the present disclosure.

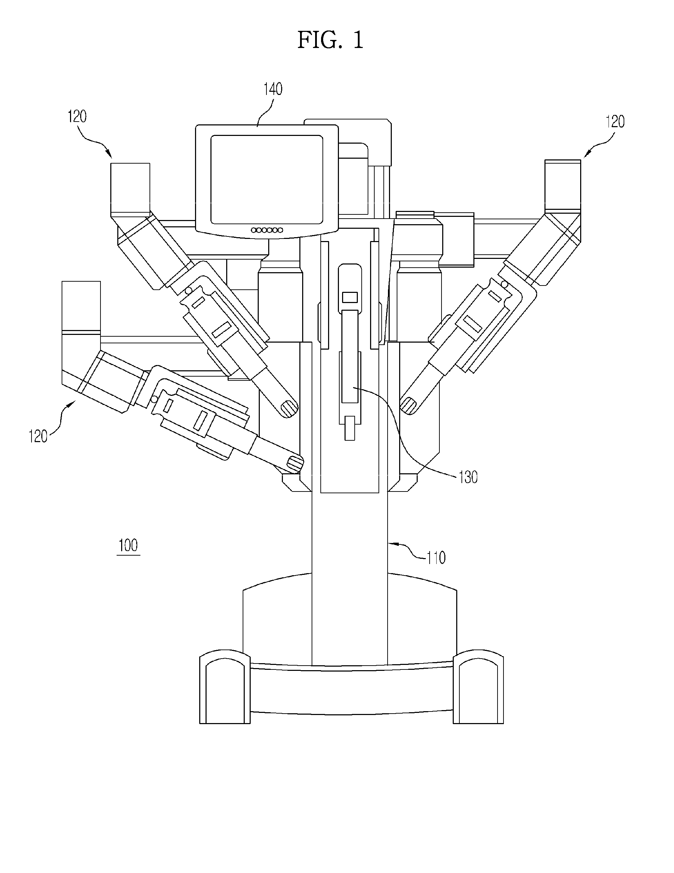

Referring to Figures 1 to 3, a surgical robot includes a manipulator assembly 100 installed at or near a surgical table or site, and a console 200 for a user to make an observation of an affected area and to control the manipulator assembly 100 to perform an operation.

As illustrated in FIG. 1, the manipulator assembly 100 includes a body 110, a plurality of surgical arms 120 movably installed at the body 110 and configured to have a surgical instrument 150 detachably coupled thereto, an endoscopy-purpose arm 130 movably installed at the body 110 and configured to obtain an image of an affected area and a surrounding of the affected area using a camera with lighting provided, and a display unit 140 configured to display an image of an affected area obtained through the endoscopy-purpose arm 130. Here, it is noted that the manipulator assembly 100 may be positioned in a surgery site as needed to perform a desired operation. For example, the manipulator assembly 100 may be portable, may be fixed, or may be detachably disposed to a site (e.g., the railing of an operating table, or other object).

The manipulator assembly 100 communicates with the console 200, and further includes a control apparatus 160 configured to control the operation of the plurality of surgical arms 120 and the endoscopy-purpose arm 130. Communication between the console 200 and control apparatus 160 may be performed over a wired or wireless network, or a combination thereof, for example.

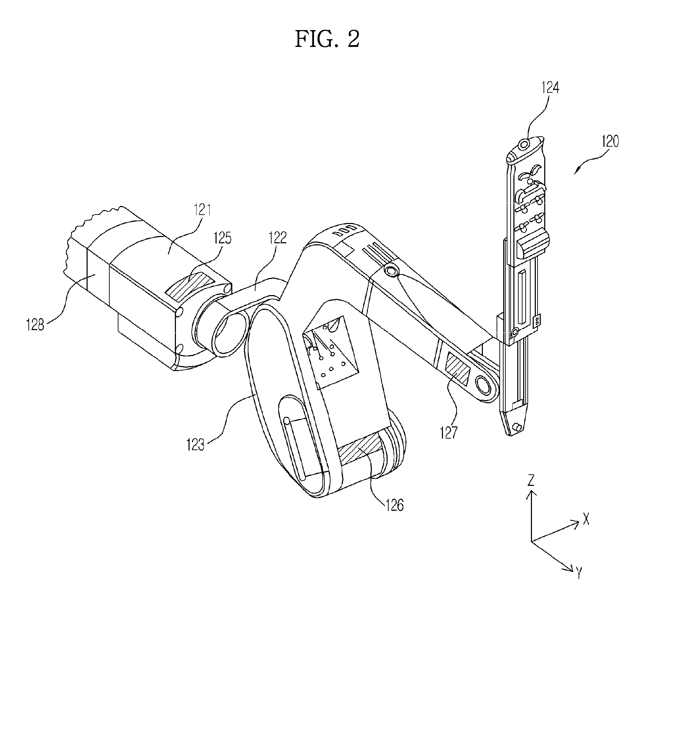

Referring to FIG. 2, the plurality of surgical arms 120 and the endoscopy-purpose arm 130 may include a plurality of links 121, 122, 123, 124. Here, links adjacent to each other among the links 121, 122, 123, 124 may be connected by a joint, and a motor may be provided at the joint.

That is, each arm may include a first link 121 connected to the body 110, a second link 122 connected to the first link 121 through the joint, a third link 123 connected to the second link 122 through the joint, and a fourth link 124 connected to the third link through the joint.

Here, the second link 122, the third link 123, and the fourth link 124 rotate on the axis of the first link 121 (Y axis), the third 123 and the fourth link 124 rotate on the axis of the second link 122 (X axis), and the fourth link 124 moves with respect to the axis of the third link 123 (X axis).

In addition, the fourth link 124 may be installed at the third link 123 for the fourth link 124 to rotate on the axis of the third link 123 (X axis).

Here, the surgical instrument 150 may be detachably installed at the fourth link 124. At this time, the surgical instrument 150 coupled to the fourth link 124 may be electrically connected to the control apparatus 160.

Each of the arms 120 and 130 may include a first motor 125, a second motor 126, and a third motor 127 configured to apply the moving force to each link so that each link may be moved, and thus, the plurality of arms may be freely moved, thereby delivering the hand movements of a user delicately and precisely. A user (e.g., a medical professional such as a physician) may remotely control the operation of the surgical robot (e.g., the arms and surgical instruments attached thereto). Generally, the user may be a physician. However, the user need not necessarily be a physician, although it would be expected that the user is a qualified or authorized operator, e.g, a medical professional. However, broadly, the user may be any user who controls the operation of the surgical robot.

In detail, each arm includes the first motor 125 installed inside the first link 121 which is configured to rotate the link 122, the link 123, and the link 124, a second motor 126 installed inside the second link 122 which is configured to rotate the third link 123 and the fourth link 124, and the third motor 127 installed inside the third link 123 which is configured to vertically move the fourth link 124.

That is, the plurality of surgical-purpose arms 120, by rotating each motor according to the command of the console 200, moves toward the direction of the multiple axes, the surgical instrument 150, by rotating each motor according to the command transmitted from the console 200, drives an end effector while moving toward the direction of the multiple axes, and the endoscopy-purpose arm 130, by rotating each motor according to the command of the console 200, moves toward the direction of the multiple axes.

That is, the plurality of surgical arms 120 and the endoscopy-purpose arm 130 of the manipulator assembly 100 may be moved by a teleoperation mode.

In addition, the plurality of surgical arms 120 and the endoscopy-purpose arm 130 may be manually moved by a user.

That is, the plurality of surgical arms 120 and the endoscopy-purpose arm 130 may be moved by a manual mode when the plurality of surgical arms 120 and the endoscopy-purpose arm 130 are manually moved by a user.

The manipulator assembly 100 may further include a torque detection unit 161 installed at a connecting portion 128 between each arm and the body 110 and configured to detect the external force applied to each arm.

The torque detection unit 161 may be installed at a link of the arm in addition to the connecting portion 128 to the body 110.

Here, the torque detection unit 161, by using the multi-axis force and torque sensor, may be able to detect the three direction elements of the external force and the three direction elements of the moment that are delivered to the arm. Here, the external force may refer to the force of a user applied to the plurality of surgical arms 120 and the endoscopy-purpose arm 130.

Each of the plurality of surgical arms 120 and the endoscopy-purpose arm 130 may further include an angle detection unit 162 configured to detect the rotational angle of the first motor 125, the second motor 126, and the third motor 127.

The angle detection unit 162 may include one or more encoders, including for example, an incremental encoder, an absolute encoder, a magnetic encoder, or a potentiometer.

The display unit 140 may be an apparatus provided for an assistant rather than for a physician, and outputs the surgical or treatment image of an affected area in two dimensions or in three dimensions. However, the display unit 140 may be used by any user and need not be provided to an assistant or physician specifically.

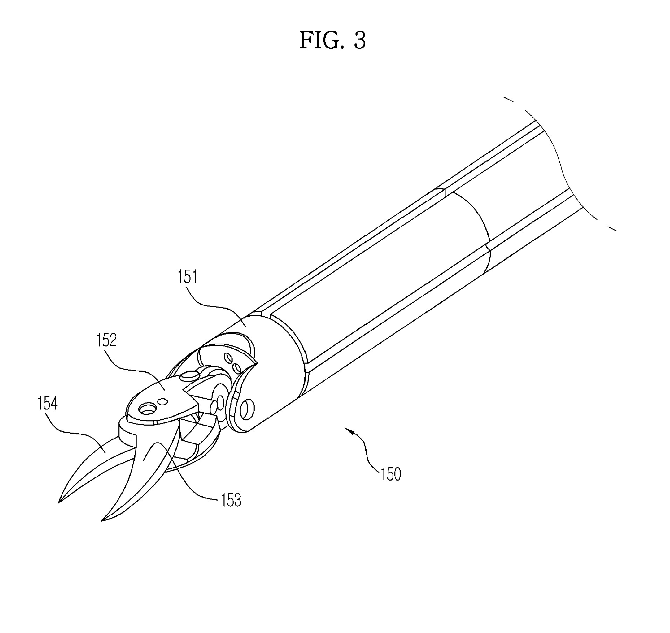

Referring to FIG. 3, the surgical instrument 150 includes a first link 151, a second link 152 connected to the first link 151 through a joint, and end effectors 153 and 154 that are connected to the second link 152 through a joint to perform a treatment or a surgery while making contact with an affected area.

The end effectors 153 and 154 rotate on the joint axis between the first link 151 and the second link 152, rotate on the joint axis between the second link 152 and the end effector 153 and the end effector 154, and rotate on the axis of the first link 151 (Z axis).

The end effectors 153 and 154 have an operating range.

The operation of the end effectors 153 and 154 may take place through a cable inside the first link 151. The cable transmits the electric signal delivered through the arm.

The end effectors 153 and 154 may include one or more devices, including for example, scissors, a grasper, a needle holder, a micro-dissector, a staple applier, a tacker, a suction irrigation tool, a clip applier, a cutting blade, an irrigator, a catheter, and a suction orifice.

In addition, the end effector of the surgical instrument may be provided with one of the probes of an electric surgery configured for ablating, resecting, cutting, and coagulating of a tissue.

A motor (not shown) may be provided at the joint connecting the adjacent links of the surgical instrument 150. That is, the end effector may be configured to rotate toward the direction of multiple axes by using the motor of the joint.

The surgical instrument 150 may further include a sensor such as a strain gauge. By conveying the data sensed at the strain gauge to the console, and then to the input unit of which a user controls, a user may be able to directly feel the pressure generated at the surgical instrument 150.

The manipulator assembly 100 further includes a button or a switch provided on the body 110, and may be directly input with the operation command from an assistant or other user.

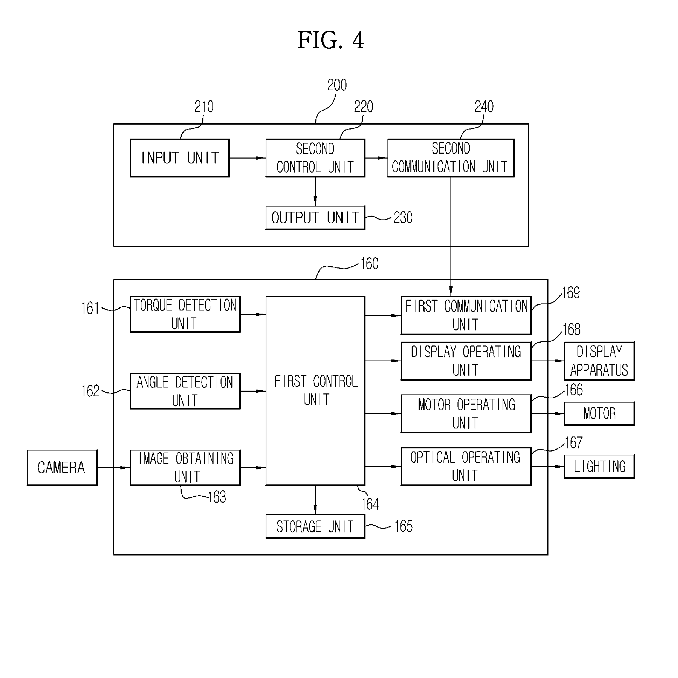

FIG. 4 shows a control block diagram illustrating the surgical robot in accordance with the embodiment of the present disclosure. For example, FIG. 4 shows the control block diagram of the control apparatus 160 of the manipulator 100 and the console 200.

The control apparatus 160 of the manipulator assembly 100 may include the torque detection unit 161, the angle detection unit 162, an image obtaining unit 163, a first control unit 164, a storage unit 165, a motor operating unit 166, an optical operating unit 167, a display operating unit 168, and a first communication unit 169.

The torque detection unit 161 may be provided at each of the plurality of arms and detects the external force applied to each arm.

The angle detection unit 162 may be provided at each of the motors of the plurality of arms and detects the rotational angle of each motor.

The image obtaining unit 163 obtains images collected from the camera inside an endoscopy. Here, a single camera may be provided in order to collect a two dimensional image (2D), or two cameras may be provided in order to obtain a three dimensional image (3D).

The image obtaining unit 163 may process the image obtained.

The first control unit 164 controls the movement of the plurality of surgical arms 120 and the endoscopy-purpose arm 130 on the basis of the external force applied by a user or the command transmitted from the console 200.

Here, a mode configured to control the arm based on the external force applied by a user is referred to as a manual mode. A mode configured to control the arm based on the command transmitted from the console 200 is referred to as a teleoperation mode.

In particular, the manual mode, after creating a posture of the arm in a range of an optimum operating angle, may be configured to control the torque of at least one of the plurality of the motors provided at the plurality of arms based on the external force applied by a user in order to maintain the posture created. The teleoperation mode may be configured to control the position, that is, the angle, of at least one of the plurality of the motors provided at the plurality of arms based on the command transmitted from the console 200.

That is, the first control unit 164, according to the teleoperation mode and the manual mode, selectively controls the operation of the endoscopy-purpose arm 130 which is configured to observe the affected area of a patient, and selectively controls the operation of the surgical instrument 150 and the plurality of surgical arms 120 which are configured to perform a treatment or a surgery on the affected area of a patient.

The first control unit 164 calculates a torque corresponding to the external force detected through the torque detection unit 161 during the manual mode, and based on the torque calculated, controls a current applied to the motor.

The first control unit 164 generates a position command based on a command transmitted from the console 200 during the teleoperation mode, calculates an angle corresponding to the position command generated, and based on the angle calculated, controls a current applied to the motor.

The control unit 164 determines whether the mode is to be changed between the manual mode and the teleoperation mode, and if determined that the mode needs to be changed, changes output data of the motor in the first mode, which corresponds to before the mode is changed, to input data of the motor in the second mode, which corresponds to after the mode is changed, and based on the input data that is set, controls the execution of the mode changed.

In particular, the first control unit 164, at the point of time when a command is transmitted from the console 200 while controlling the motor in the manual mode, determines that the mode needs to be changed from the manual mode to the teleoperation mode, checks an angle detected through the angle detection unit 162 at the time of when the manual mode is stopped, sets the angle checked as an initial angle of the teleoperation mode, and controls the change of the angle of the motor from the initial angle to an angle corresponding to the command transmitted from the console 200.

The first control unit 164, in order to improve the characteristic of the response of the position control, controls the speed of the motor at the time of changing the angle.

The first control unit 164, when it is determined that the external force is applied to the arm while controlling the motor in the teleoperation mode, determines that the mode needs to be changed from the teleoperation mode to the manual mode, checks a torque generated at the time when the teleoperation mode is stopped, sets the torque checked as an initial torque of the manual mode, and controls the torque of the motor from the initial torque set to a torque corresponding to the external force.

At this time, the first control unit 164 controls the torque of the motor by executing interpolation.

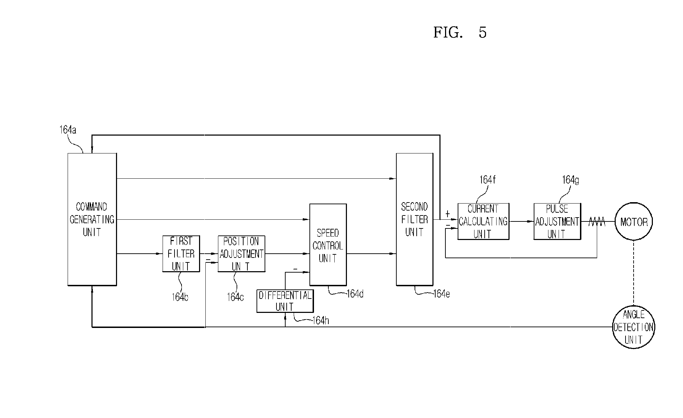

Referring to FIG. 5, the control structure of the motor configured to change the mode using the first control unit 164 will be explained hereafter.

The first control unit 164 configured to control the motor to change the mode may include a command generating unit 164a, a first filter unit 164b, a position adjustment unit 164c, a speed control unit 164d, a second filter unit 164e, a current calculating unit 164f, a pulse adjustment unit 164g, and a differential unit 164h.

The command generating unit 164a, if an external force is detected in the manual mode, calculates a torque corresponding to the external force, and outputs a torque command by generating a command to follow the torque calculated. The command generating unit 164a outputs a position command by generating a command to follow a position corresponding to a command transmitted from the console in the teleoperation mode.

The command generating unit 164a, when the mode is changed from the manual mode to the teleoperation mode, detects the angle of the motor at the time the mode is changed, sets the angle detected as an initial angle of the motor in the teleoperation mode, and outputs a position command to follow the initial angle set. The command generating unit 164a, when the mode is changed from the teleoperation mode to the manual mode checks a torque of the motor, sets the torque checked as an initial torque in the manual mode, and outputs a torque command to follow the initial torque set.

The first filter unit 164b filters the position command generated from the command generating unit 164a.

The position adjustment unit 164c calculates a speed command based on the position output from the command generating unit 164a and the actual position of the motor, outputs the speed command calculated, and controls the position of the motor so that the two position signals coincide to each other.

The speed control unit 164d calculates and outputs a torque command based on the speed command output from the position adjustment unit and the actual speed output from the differential unit 164h, and controls the speed of the motor so that the two speed signals coincide to each other.

Thus, by reflecting the actual position and the actual speed, the responsiveness and the ability in the following of the torque may be improved.

The second filter unit 164e may be a filter installed to eliminate a resonance or a vibration caused by the natural vibration frequency of the link that forms the arm, and may be configured to filter the torque command and output the torque command filtered.

The torque command output from the second filter unit 164e is entered to the current calculating unit 164f and the command generating unit 164a.

The current calculating unit 164f, when the torque command is input from one of the speed control unit and the command generating unit, calculates the current to follow the torque being input. In addition, a current feedback signal of the current that flows at the motor in practice is input to the current calculating unit 164f.

The current calculating unit 164f, by comparing the current to follow the torque with the actual current, calculates the current that is to be applied to the motor, and outputs to the pulse adjustment unit 164g.

The pulse adjustment unit 164g may be a current conversion apparatus and be configured to adjust the pulse width so that the current calculated is generated, and thereby the electrical power may be converted by the pulse width that is adjusted. The electrical power converted hereby is applied to the motor.

The differential unit 164h may be a differentiator configured to differentiate the angle detected from the angle detection unit to detect the actual speed. The differential unit 164h outputs the actual speed detected to the speed control unit.

The motor may be a servo motor provided at the arm and configured to move the link.

The angle detection unit may be an encoder configured to detect the rotational position, for example, a rotational angle of the servo motor, and is configured to detect the rotational position of the rotation axis in connection with the rotation axis of the servo motor.

The angle of the motor detected at this time is output to the command generating unit and the position adjustment unit.

Thus, when controlling the position of the motor, the torque command output from the second filter unit 164e is input again to the command generating unit, and when controlling the torque of the motor, the actual angle detected by the angle detection unit is input again to the command generating unit, so that the output data between the position control and the torque control may be shared with each other.

That is, as the control structure between the position control of the motor and the torque control of the motor is connected in parallel, the output data may be shared with each other. Thus, the motor may be stably controlled.

The storage unit 165 stores the image of an affected area and the torque and the angle of the motor provided at each of the plurality of surgical arms 120 and the endoscopy-purpose arm 130. The storage unit may be embodied as a non-transitory computer readable medium, including hard disks, floppy disks, flash memory or memory cards (e.g., a USB drive), or optical media such as CD ROM discs and DVDs.

The motor operating unit 166 operates the motor provided at each of the plurality of surgical arms 120 and the endoscopy-purpose arm 130 and the motor provided at the surgical instrument 150 according to the command of the first control unit.

The optical operating unit 167 operates the lighting inside the endoscopy. Here, one or more lighting devices may be provided.

The display operating unit 168 operates the display apparatus 140 according to the command of the first control unit 164. The display apparatus 140 may include a liquid crystal display (LCD) or light emitting diode (LED) display, for example. However, the disclosure is not so limited and may include other types of displays.

The first communication unit 169 transmits the image of an affected area to the console 200 according to the command of the first control unit 164, and transmits the operation command of each motor received from the console 200 to the first control unit 164.

The first communication unit 169 may transmit the torque and the angle data of the each motor provided at each of the plurality of surgical arms 120 and the endoscopy-purpose arm 130 to the console 200. Communication between the first communication unit 169 of the control apparatus 160 and the console 200 may be performed over a wired or wireless network, or a combination thereof.

The console 200 may be an apparatus that enables a treatment or a surgery of an affected area by enabling the surgical instrument to make the same movement as a virtual movement of a surgical operation, as a user performs the virtual movement of a surgical operation while looking at the image of an affected area that is obtained through the endoscopy of the manipulator assembly 100.

The console 200 may include an input unit 210, a second control unit 220, an output unit 230, and a second communication unit 240.

The input unit 210 receives a command to control the plurality of surgical arms 120, the endoscopy-purpose arm 130, and the surgical instrument 150 provided at the manipulator assembly 100.

Here, the input unit 210 may include at least one of a small-size wrist gym ball, a joystick, a glove, a trigger gun, or a voice recognition apparatus.

The input unit 210 may further include foot pedals (e.g., four foot pedals) configured to guide a hemostasis or to control a vertical or a horizontal movement of the surgical instrument or the endoscopy-purpose arm. The input unit 210 enables a user (e.g., an operator) to input an operation command to control the surgical robot and may include a user interface (UI). Also, the input unit 210 may include a device such as a keyboard or mouse and may further have additional features to assist the user in operating the surgical robot, including haptic feedback capability, head-mounted displays, or virtual reality devices, for example. The input unit may be remotely located from the manipulator assembly 100 and plurality of surgical arms 120 such that a user may input an operation command from a remote station.

The second control unit 220 controls the transmission of the operation command of the motor provided at the arm corresponding to the command input through the input unit 210, the transmission of the operation command of the surgical instrument, and the transmission of the operation command of the endoscopy.

The second control unit 220 controls the storage of the angle and the torque of the motor provided at the arm and the motor provided at the surgical instrument.

The second control unit 220, when the operation command for the motor provided at the arm, the surgical instrument, and the endoscopy is input, may check the angle and the torque of the motor, may generate a next command using the angle or the torque checked, and may control the transmission of the command generated.

The second control unit 220 controls the output of the image transmitted from the manipulator assembly 200.

The output unit 230 outputs the image of an affected area to a user. The output unit 230 includes an optical lens configured to view a stereo image.

The output unit 230 may convert and output the image of an affected area into a stereo image for a user to operate the arm, the surgical instrument, and the endoscopy while observing an affected area in real time.

The second communication unit 240 conducts a wired or wireless communication with the first communication unit 169 of the manipulator assembly 100.

The second communication unit 240 transmits the signal to control the plurality of surgical arms 120, the endoscopy-purpose arm 130, and the surgical instrument 150 provided at the manipulator assembly 100, and receives the image signal of the affected area from the manipulator assembly 100.

In addition, the second communication unit 240 may receive the angle and the torque of the motor provided at the arm of the manipulator assembly 100 and at the surgical instrument.

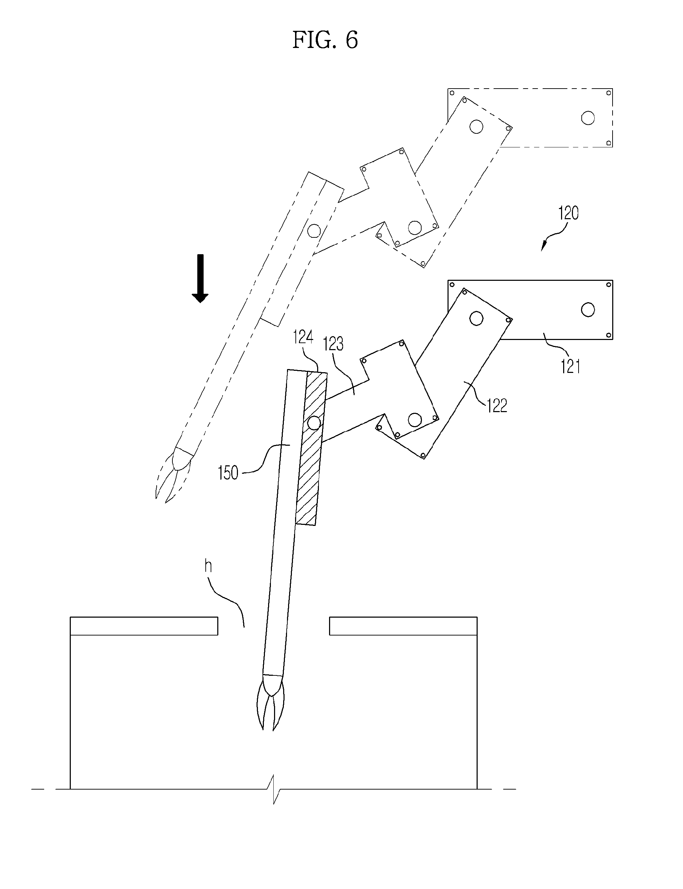

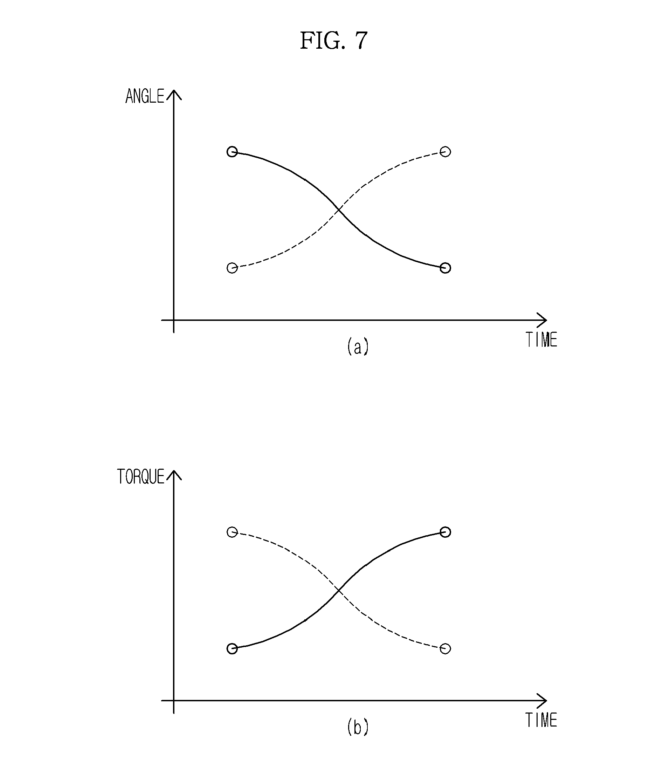

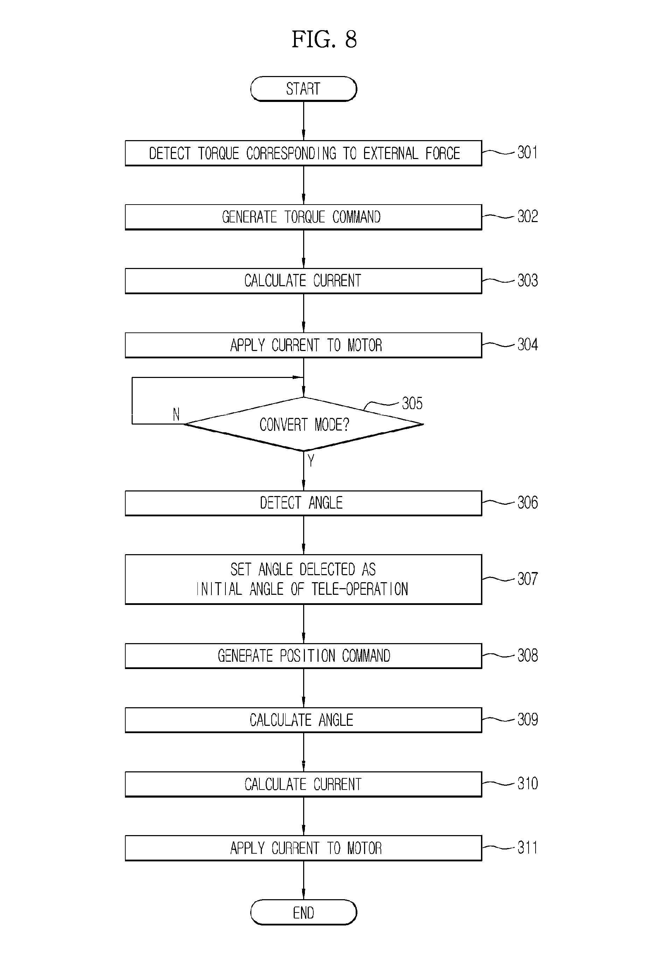

FIGS. 8 to 9 are control flow charts of a surgical robot in accordance with the embodiment of the present disclosure, and the control flow will be explained in conjunction with FIG. 6 and FIG. 7.

FIG. 8 is a control flow chart showing the mode change from the manual mode to the teleoperation mode.

Referring to FIG. 6 and FIG. 8, a medical professional (e.g., a physician or an assistant) forms an incision (h) creating a small puncture on the skin covering an affected area of a patient or subject, moves the plurality of surgical arms 120 and the endoscopy-purpose arm 130 to the position of the incision (h) by applying an external force to each of the plurality of surgical arms 120, and inserts the endoscopy and the plurality of surgical arms 120 including one or more corresponding instruments into the incision (h).

At this time, the manipulator assembly of the surgical robot controls the operation of the motor provided at each arm in the manual mode.

The following description will be made in relation to one of the plurality of motors as an example.

First, the control apparatus 160 inside the manipulator assembly 100 of the surgical robot detects the external force applied to the arm (301). At this time, the external force is detected by the torque detection unit 161 provided at the arm.

Next, the manipulator assembly of the surgical robot generates the torque command of the motor to move the arm smoothly to correspond to the external force (302).

Next, the manipulator assembly of the surgical robot calculates the current to follow the torque calculated (303), and converts the electrical power by adjusting the pulse width to correspond to the current calculated.

Here, when calculating the current to follow the torque calculated, the actual current provided to the motor may be fed back and may be reflected in the calculation of the current.

Next, the manipulator assembly of the surgical robot, by applying the electrical power converted to the motor (304), enables a torque that corresponds to the torque command to be generated at a corresponding motor.

Next, the manipulator assembly of the surgical robot determines the point in time needed to change the mode (305). Here, the determining of the point in time needed to change the mode refers to the determining of whether the operation command of the arm is input from the console 200.

Next, the manipulator assembly of the surgical robot, at the time of when a command is transmitted from the console, determines that the mode needs to be changed from the manual mode to the teleoperation mode, and detects the angle of the motor at the point in time when the manual mode is stopped (306).

Next, the manipulator assembly of the surgical robot sets the angle detected as the initial angle of the teleoperation mode, and conducts the position control in changing the angle of the motor from the initial angle to an angle corresponding to the command transmitted from the console 200.

Referring to FIG. 7, part (a), by smoothly changing the angle of the motor from the initial angle to the angle corresponding to the command transmitted regardless of the increase or decrease of the angle, the motor may be stably controlled.

Next, the manipulator assembly of the surgical robot, while generating the position command of the motor corresponding to the command transmitted from the console 200, conducts the teleoperation mode that controls the movement of the arm from a remote position.

Here, the conducting of the teleoperation mode refers to the generating of the position command based on the command transmitted from the console 200 (308), the calculating of the angle corresponding to the position command generated (309), and the generating of the speed command corresponding to the angle calculated. At this time, the speed command may be directly input from the command generating unit.

Next, the manipulator assembly of the surgical robot generates the torque command corresponding to the speed command, and calculates the current configured to follow the torque command generated (310).

Alternatively, an angle of the motor may be detected, an actual speed is calculated by differentiating the angle detected, and a torque command is generated by reflecting the actual speed calculated in a speed command.

In addition, in a case when calculating the current to follow the torque command, the actual current applied to the motor may be fed back and reflected in the calculation of the current.

Next, the manipulator assembly of the surgical robot, by adjusting the pulse width to correspond to the current calculated, converts the electrical power.

Next, the manipulator assembly of the surgical robot, by applying the electrical power converted to the motor (311), enables a torque corresponding to the position command to be generated at a corresponding motor.

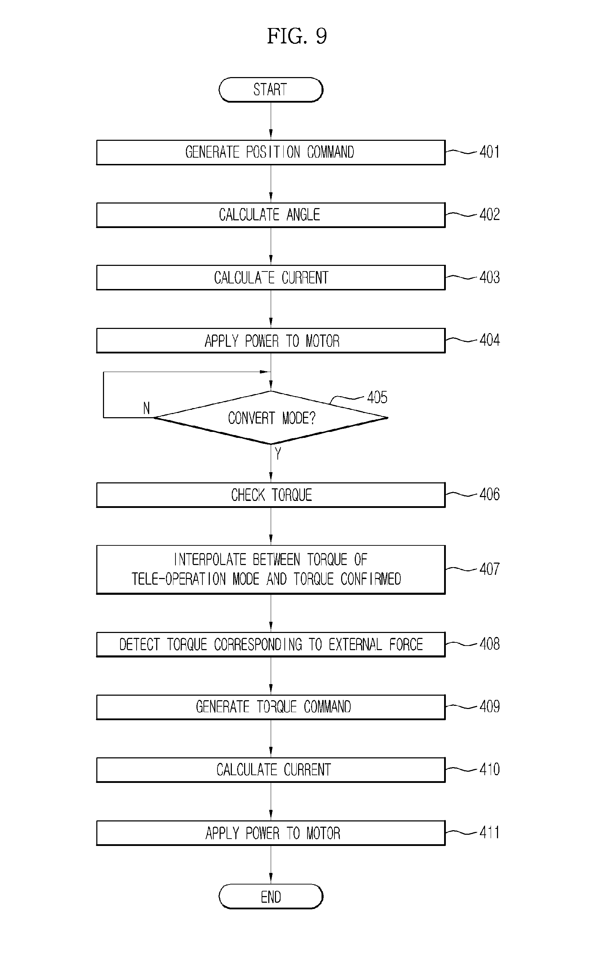

FIG. 9 is a control flow chart illustrating the mode changing from the teleoperation mode to the manual mode.

The surgical robot, based on the command transmitted from the console, by conducting the teleoperation mode configured to control the position of the motor, performs a treatment or a surgery on an affected area.

Here, the conducting of the teleoperation mode refers to the generating of a position command based on the command transmitted from the console 200 (401), the calculating of an angle corresponding to the position command generated (402), and the generating of a speed command corresponding to the angle calculated. At this time, the speed command may be directly input from the command generating unit.

Next, the manipulator assembly of the surgical robot generates a torque command corresponding to the speed command, and calculates a current to follow the torque command generated (403).

Alternatively, an angle of the motor may be detected, an actual speed is calculated by differentiating the angle detected, and the calculated actual speed is reflected in a speed command, thereby generating a torque command.

In addition, in a case when calculating the current to follow the torque command, the actual current applied to the motor may be fed back so as to be reflected in the calculation.

Next, the manipulator assembly of the surgical robot, by adjusting the pulse width to correspond to the current calculated, converts the electrical power.

Next, the manipulator assembly of the surgical robot, by applying the electrical power converted, enables a torque corresponding to the position command to be generated at a corresponding motor (404).

That is, the manipulator assembly of the surgical robot, in a case of controlling in the teleoperation mode, operates the motor to move the arm to a target position based on the position command, and the torque command, and enables the arm to move to the target position at a smoother and more precise manner by further reflecting the speed command, hereby improving the responsiveness of the position control.

Next, the manipulator assembly of the surgical robot determines whether the mode needs to be changed from the teleoperation mode to the manual mode (405).

That is, in a case when a treatment or a surgery is completed while the motor is controlled at the teleoperation mode or when the position of the arm needs to be changed manually, the change of the mode from the teleoperation mode to the manual mode takes place.

The manipulator assembly of the surgical robot, at the point in time of when the external force from outside is detected at the arm while conducting the teleoperation mode, determines that the mode needs to be changed from the teleoperation mode to the manual mode, checks the torque of the motor at the time of when the conducting of the teleoperation mode is stopped, and sets the torque checked as the initial torque of the manual mode.

By performing an interpolation operation from the initial torque of the manual mode to the torque corresponding to the external force (407), the torque of the motor may be controlled.

Referring to part (b) of FIG. 7, by smoothly changing the torque of the motor from the initial torque to the torque corresponding to the external force regardless of the increase or decrease of the torque, the motor may be stably controlled.

Next, the manipulator assembly of the surgical robot periodically detects the torque corresponding to the external force (408), and generates the torque command of the motor for the arm to move smoothly to correspond to the external force (409).

Next, the manipulator assembly of the surgical robot calculates the current to follow the torque calculated (410), and by adjusting the pulse width to correspond to the current calculated, converts the electrical power.

Here, in a case when calculating the current to follow the torque calculated, the actual current provided to the motor may be fed back and reflected in the calculation.

Next, the manipulator assembly of the surgical robot, by applying the electrical power converted to the motor (411), enables a torque corresponding to the torque command to be generated at a corresponding motor.

As described above, in a case when the position is controlled in the teleoperation mode and the torque is controlled in the manual mode, as the final output is shared between each operation mode, a stable conversion of the control during the change of the mode may be possible.

In addition, with respect to the arm of the surgical robot, the vibration and the abrupt change of the posture which may occur during the change of the mode between the manual mode and the teleoperation mode may be restrained, thereby increasing stability of the surgical robot.

The components constituting the surgical robot including the manipulator assembly 100, including the surgical arms 120, surgical instrument 150, and control apparatus 160, and a console 200 and corresponding methods for controlling the components according to the above-described example embodiments, may use one or more processors, which may include a microprocessor, central processing unit (CPU), digital signal processor (DSP), or application-specific integrated circuit (ASIC), as well as portions or combinations of these and other processing devices.

The disclosure herein has provided example embodiments of a surgical robot including the manipulator assembly 100, including the surgical arms 120, surgical instrument 150, and control apparatus 160, and a console 200 and control methods thereof, which may be applied for example, in a medical setting to perform an operation on a patient (e.g., a human or animal or other lifeform). However, the disclosure is not so limited. For example, the surgical robot may be used in other settings which may benefit from the example embodiments disclosed herein. For example, the surgical robot may be utilized to perform operations in any confined space or enclosure in which an operator may need to perform controlled movements using an instrument attached to a robot arm, so as to avoid or to prevent injuries to bodies or objects, that may be located or disposed within the space or enclosure, due to imprecise movements of the surgical robot. Possible settings may include, for example, mining operations, surveillance operations, inspection operations, repair operations, bomb disposal operations, etc., however again, the disclosure is not so limited.

The terms "module", and "unit," as used herein, may refer to, but are not limited to, a software or hardware component or device, such as a Field Programmable Gate Array (FPGA) or Application Specific Integrated Circuit (ASIC), which performs certain tasks. A module or unit may be configured to reside on an addressable storage medium and configured to execute on one or more processors. Thus, a module or unit may include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. The functionality provided for in the components and modules/units may be combined into fewer components and modules/units or further separated into additional components and modules.

The methods for controlling the surgical robot according to the above-described example embodiments may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM discs and DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described embodiments, or vice versa. Some or all of the operations performed in the methods for controlling the surgical robot according to the above-described example embodiments may be performed over a wired or wireless network.

Each block of the flowchart illustrations may represent a unit, module, segment, or portion of code, which comprises one or more executable instructions for implementing the specified logical function(s). It should also be noted that in some alternative implementations, the functions noted in the blocks may occur out of the order. For example, two blocks shown in succession may in fact be executed substantially concurrently or the blocks may sometimes be executed in the reverse order, depending upon the functionality involved.

Although a few example embodiments of the present invention have been shown and described, it would be appreciated by those skilled in the art that changes may be made to these embodiments without departing from the principles of the invention, the scope of which is defined in the claims.

## Claims

1. A surgical robot having a console and a manipulator assembly, wherein the manipulator assembly comprises:
at least one arm having a plurality of links and a motor provided between adjacent ones of the links; and
a control unit configured to determine whether a mode is to be changed from a first mode to a second mode; and
if it is determined that the mode is to be changed, the control unit is configured to determine a motor parameter from the first mode and to set the determined parameter as an initial parameter to control the motor in the second mode.

2. The surgical robot of claim 1, wherein the first mode is one of a teleoperation mode for controlling the motor based on a command transmitted from the console and a manual mode for controlling the motor based on an external force, and the second mode is the other of the teleoperation mode and the manual mode.

3. The surgical robot of claim 2, wherein:
the manipulator assembly further comprises a torque detection unit configured to detect an external force applied to the at least one arm, and
the control unit is configured to operate in the manual mode if the external force is detected.

4. The surgical robot of claim 3, wherein:
the control unit, in the case of controlling the motor in the manual mode, is configured to calculate a torque corresponding to the external force, and to control a current applied to the motor based on the calculated torque.

5. The surgical robot of claim 2, 3 or 4, wherein:
the manipulator assembly further comprises an angle detection unit configured to detect an angle of the motor, and
the control unit, when the command is transmitted from the console during the manual mode, is configured to change the manual mode to the teleoperation mode, and to set the angle detected as an initial angle of the teleoperation mode.

6. The surgical robot of claim 5, wherein:
the control unit is configured to control a change of the angle of the motor from the initial angle to an angle corresponding to the command transmitted from the console.

7. The surgical robot of any one of claims 2 to 6, wherein:
the control unit, in the case of controlling the motor in the teleoperation mode, is configured to generate a position command based on the command transmitted from the console, to calculate an angle corresponding to the position command generated, and to control a current applied to the motor based on the calculated angle.

8. The surgical robot of claim 7, wherein:
the control unit is configured to control a speed of the motor when the current applied to the motor is controlled based on the calculated angle.

9. The surgical robot of any one of claims 2 to 8, wherein:
if it is determined that the mode is changed from the teleoperation mode to the manual mode, the control unit is configured to check a torque of the motor provided when the motor is controlled in the teleoperation mode, and to set the torque checked as an initial torque of the manual mode.

10. The surgical robot of claim 9, wherein:
the control unit is configured to calculate a torque corresponding to the external force when the mode is changed from the teleoperation mode to the manual mode, and
to control the torque of the motor from the initial torque to the calculated torque.

11. The surgical robot of claim 10, wherein:
the control unit is configured to control the torque of the motor by performing interpolation.

12. The surgical robot of any of claims 2 to 11, wherein the control unit comprises:
a command generating unit configured to generate a position command and a torque command;
a position adjusting unit configured to adjust the angle of the motor in response to the position command generated;
a current producing unit configured to calculate a current used to follow a torque corresponding to the generated torque command and to generate a current used to adjust the angle of the motor; and
an electrical power converting unit configured to adjust a pulse applied to the motor based on the calculated current.

13. A method of controlling a surgical robot having a console and a manipulator assembly, the method comprising:
detecting an external force applied to an arm provided at the manipulator assembly;
performing a manual mode configured to control a torque of the motor based on the external force detected;
determining whether a command is transmitted from the console;
determining a point in time to change from the manual mode to the teleoperation mode when it is determined the command is transmitted from the console;
detecting an angle of the motor;
setting the detected angle as an initial angle of the teleoperation mode;
controlling a change of the angle of the motor from the initial angle to an angle corresponding to the command transmitted; and
controlling the teleoperation mode.

14. The method of claim 13, wherein the performing of the manual mode comprises:
generating a torque command corresponding to the external force;
calculating a current to follow the generated torque command; and
adjusting a pulse width of the current and applying a current to the motor reflecting the adjusted pulse width;
and/or wherein the performing of the teleoperation mode comprises:
generating a position command based on the command transmitted from the console;
calculating an angle corresponding to the position command generated;
calculating a current corresponding to the calculated angle, and
adjusting a pulse width of the current and applying a current to the motor reflecting the adjusted pulse width.

15. The method of claim 14, further comprising:
determining a point in time to change from the teleoperation mode to the manual mode when the external force applied to the arm is detected during the execution of the teleoperation mode,
checking the torque of the motor at the time of the completion of the execution of the teleoperation mode,
setting the torque checked as an initial torque of the manual mode, and
controlling the torque of the motor from the initial torque to a torque corresponding to the external force.
